# EUROPÄISCHE PATENTSCHRIFT

(11) **EP 2 412 315 B1**
(45) Veröffentlichungstag und Bekanntmachung des Hinweises auf die Patenterteilung: **03.04.2019**
(21) Anmeldenummer: 11175029.5
(22) Anmeldetag: 22.07.2011
(51) Int. Cl.: A61B 17/00

(54) **Vorrichtung zum Aufbringen einer Ummantelung auf das distale Ende eines chirurgischen Schaftinstruments**
Device for applying a cladding to the distal end of a surgical shaft instrument
Dispositif d'application d'une gaine sur l'extrémité distale d'un instrument chirurgical à tige

(30) Priorität: 28.07.2010 DE 102010032606
(43) Veröffentlichungstag der Anmeldung: 01.02.2012
(73) Patentinhaber: Karl Storz SE & Co. KG, 78532 Tuttlingen (DE)
(72) Erfinder: Blocher, Martin, 78532 Tuttlingen (DE); Merz, Robin, 78532 Tuttlingen (DE)

(56) Entgegenhaltungen:
- US-A- 5 564 431
- US-A- 6 049 960
- US-A1- 2007 149 850
- US-A1- 2009 234 374

## Beschreibung

Die Offenbarung betrifft ein Verfahren zum Aufbringen einer Ummantelung auf ein distales Ende eines chirurgischen Schaftinstruments, insbesondere die Werkzeugspitze eines Dilatationsinstruments.

Die Erfindung betrifft eine Vorrichtung zur Durchführung dieses Verfahrens.

Dilatationsinstrumente werden beispielsweise in der endoskopischen HNO-Chirurgie verwendet, um die Stirnhöhle eines Patienten zu erweitern und knöcherne Verwachsungen in der Stirnhöhle zu entfernen.

Um das Eindringen von Gewebe in die Dilatationsmechanik zu verhindern und die Rückstellung der Segmente der Dilatationsmechanik zu ermöglichen, ist über das mit dem Dilatationswerkzeug versehene distale Ende des Schaftinstruments eine Ummantelung ziehbar. Aufgrund der kleinen Baugröße der endoskopischen Instrumente ist es sehr schwierig die Ummantelung manuell über die distale Instrumentenspitze zu ziehen und gleichzeitig darauf zu achten, dass die Ummantelung bei diesem Aufziehen nicht beschädigt wird, da die Werkzeuge an der Instrumentenspitze häufig scharfkantige Abschnitte aufweisen.

Die US-Patentanmeldung 2007/0149850 A1 lehrt ein Werkzeugset zum Aufbringen einer Endkappe auf das distale Ende eines Endoskops. Die Endkappe ist elastisch dehnbar und weist eine hohlzylindrische Form auf. Ihr Innendurchmesser kleiner ist als der Außendurchmesser des distalen Abschnitts des Endoskops. Das Werkzeugset umfasst drei Komponenten, die sich konzentrisch zusammenstecken lassen. Die elastische Endkappe wird auf ein Aufziehteil gezogen und aufgeweitet, indem ein Aufweitkolben mit konischer Spitze in das Aufziehteil eingeführt wird. Das Aufziehteil kann nunmehr auf das distale Ende des Endoskops gesetzt und die Endkappe im aufgeweiteten Zustand mittels eines Halteteils vom Aufziehteil auf das distale Ende des Endoskops gezogen werden. Das genannte Werkzeugset setzt einen beidseitig geöffneten Überzug voraus und ist nicht für einen sackartigen, nur einseitig geöffneten Überzug geeignet.

Davon ausgehend liegt der Offenbarung die **Aufgabe** zugrunde, ein Verfahren zum Aufbringen einer Ummantelung auf das distale Ende eines chirurgischen Schaftinstruments zu schaffen, das bei einfacher Handhabung ein schnelles und materialschonendes Festlegen der Ummantelung auf der Instrumentenspitze ermöglicht. Weiterhin liegt der Erfindung die **Aufgabe** zugrunde, eine Vorrichtung zur Durchführung des Verfahrens bereitzustellen.

Die **Lösung** der verfahrensmäßigen Aufgabenstellung ist gekennzeichnet, durch die Verfahrensschritte:
a) Festlegen der Ummantelung an einer Vorrichtung zum Aufbringen einer Ummantelung auf das distale Ende eines chirurgischen Schaftinstruments,
b) radiales Aufweiten zumindest des proximalen Endes der Ummantelung und Einführen des distalen Endes des chirurgischen Schaftinstruments in die aufgeweitete Ummantelung,
c) Übergabe der Ummantelung von der Vorrichtung zum Aufbringen einer Ummantelung auf das distale Ende eines chirurgischen Schaftinstruments an das distale Ende des chirurgischen Schaftinstruments und
d) Entnehmen des mit der Ummantelung versehenen distalen Endes des chirurgischen Schaftinstruments aus der Vorrichtung zum Aufbringen einer Ummantelung auf das distale Ende eines chirurgischen Schaftinstruments.

Das offenbarte Verfahren ermöglicht ein einfaches Aufziehen der Ummantelung auf die Instrumentenspitze bei gleichzeitiger Schonung des Materials der Ummantelung. Das radiale Aufweiten der Ummantelung im Verfahrensschritt b) ermöglicht ein einfaches Einführen der Instrumentenspitze in die Ummantelung, ohne die Gefahr, das Material der Ummantelung mit der Instrumentenspitze zu beschädigen.

Gemäß einer bevorzugten Ausführungsform der Offenbarung wird vorgeschlagen, dass das radiale Aufweiten des proximalen Endes der Ummantelung im Verfahrensschritt b) direkt oder indirekt durch das distale Ende des chirurgischen Schaftinstruments erfolgt.

Die Erfindung wird durch den unabhängigen Anspruch 1 definiert. Die abhängigen Ansprüche beschreiben weitere vorteilhafte Ausführungsformen.

Die **Lösung** der vorrichtungsmäßigen Aufgabenstellung ist erfindungsgemäß gekennzeichnet, durch einen mindestens zwei Spreizelemente aufweisenden Grundkörper, wobei die Ummantelung an den Spreizelementen festlegbar ist und zwischen den Spreizelementen eine Einführöffnung zur Aufnahme des distalen Endes des chirurgischen Schaftinstruments ausgebildet ist. Durch das Festlegen der Ummantelung an den Spreizelementen der Aufziehvorrichtung bedarf es keiner weiteren manuellen Betätigung, um die Ummantelung auf die Instrumentenspitze zu übertragen.

Mit einer ersten Ausführungsform der Erfindung wird vorgeschlagen, dass die Spreizelemente an einem Grundkörper gelagert sind.

Gemäß der Erfindung ist vorgesehen, dass die mindestens zwei Spreizelemente radial zur Einführöffnung verlagerbar am Grundkörper gelagert sind, so dass das an den Spreizelementen festlegbare proximalseitige Ende der Ummantelung durch die Spreizelemente radial aufweitbar ist.

Mit einer praktischen Ausführungsform der Erfindung wird vorgeschlagen, dass der Grundkörper kastenförmig so ausgebildet ist, dass die Einführöffnung in einer Stirnplatte des Grundkörpers ausgebildet ist und sich die Spreizelemente von der Einführöffnung fort in das Innere des Grundkörpers erstrecken. Diese Ausgestaltungsform zeichnet sich durch eine robuste und einfach zu handhabende Bauweise aus.

Zusätzlich zur Verwendung des mindestens einen Federelements wird mit der Erfindung vorgeschlagen, dass mindestens ein Spreizelement der Aufziehvorrichtung federnd ausgebildet ist, wobei optional bei der zusätzlichen Federelastizität des mindestens einen Spreizelements diese Federelastizität durch die Dimensionierung des Spreizelements und/oder durch dessen Materialbeschaffenheit so einzustellen ist, dass die Federelastizität des wenigstens einen federnd ausgebildeten Spreizelements geringer ausgebildet ist als die Federkraft des mindestens einen Federelements.

Um einen sicheren Halt der Ummantelung auf den Spreizelementen der Aufziehvorrichtung zu gewährleisten, wird mit der Erfindung vorgeschlagen, dass am distalen Ende der Spreizelemente ein Haltewulst oder eine Halteausnehmung zum Festlegen der Ummantelung ausgebildet ist und am proximalen Ende der Ummantelung ein umlaufender Wulst ausgebildet ist, über den die Ummantelung an den Spreizelementen festlegbar ist. Der proximalseitige umlaufende Wulst der Ummantelung dient weiterhin dazu, die Ummantelung lagesicher am distalen Ende des chirurgischen Schaftinstruments festlegen zu können. Der umlaufende Wulst dient zudem als Reißschutz für den proximalen Bereich der Ummantelung.

Zur Entnahme der mit der Ummantelung versehenen Instrumentenspitze aus der Aufziehvorrichtung wird mit der Erfindung vorgeschlagen, dass in der Stirnplatte des Grundkörpers seitlich neben der Einführöffnung mindestens eine mit der Einführöffnung verbundene Entnahmeöffnung ausgebildet ist. Das Überführen der mit der Ummantelung versehenen Instrumentenspitze von der Einführöffnung in die im Durchmesser größere Entnahmeöffnung stellt sicher, dass die Ummantelung auch wieder materialschonend aus der Aufziehvorrichtung entnommen werden kann.

Vorteilhafterweise ist die erfindungsgemäße Aufziehvorrichtung selbst als Spritzgußteil ausgebildet, was eine schnelle und einfache Fertigung der Vorrichtung ermöglicht.

Mit einer weiteren Ausführungsform, die nicht unter den Schutzanspruch der Ansprüche fällt wird vorgeschlagen, dass die Spreizelemente gegeneinander verschwenkbar aneinander gelagert sind. Bei dieser Ausführungsform bedarf es keines Grundkörpers und keiner gesonderten Federelemente, weshalb diese Ausführungsform sich durch ihren einfachen Aufbau aus nur wenigen Bauteilen auszeichnet.

Gemäß einer praktischen Ausführungsform dieser weiteren Ausführungsform wird vorgeschlagen, dass die Spreizelemente lösbar aneinander gelagert sind, um eine einfache Montage und Demontage der Aufziehvorrichtung zu ermöglichen.

Mit einer bevorzugten Ausführungsform der weiteren Ausführungsform wird vorgeschlagen, dass die Aufziehvorrichtung aus zwei im Querschnitt U-förmige halbschalig ausgebildeten Spreizelementen besteht. Durch die halbschalige Ausbildung der Spreizelemente ist es möglich, mit nur zwei Spreizelementen die zu ummantelnde Werkzeugspitze vollständig zu umschließen.

Zur Ausbildung der gegenseitigen Verschwenkbarkeit der Spreizelemente wird vorgeschlagen, dass an den Spreizelementen Lagerzapfen und Lageraufnahmen ausgebildet sind. Vorteilhafterweise sind an jedem Spreizelement ein Lagerzapfen und eine Lageraufnahme ausgebildet, so dass es nur eines Spreizelementtyps bedarf, aus dem paarweise verwendet eine Aufziehvorrichtung montierbar ist.
Zur Montage der Aufziehvorrichtung sowie zum Abnehmen der Aufziehvorrichtung von der ummantelten Werkzeugspitze sind die Spreizelemente voneinander lösbar ausgebildet. Hierzu wird vorgeschlagen, dass die Lagerzapfen und Lageraufnahmen so ausgebildet sind, dass die Spreizelemente nur in einer einzigen gegeneinander verschwenkten Position voneinander lösbar sind. Vorteilhafterweise ist dies die am weitesten gegeneinander verschwenkte Position der Spreizelemente, um ein versehentliches Lösen der Spreizelemente während der Ummantelungsphase zu vermeiden.
Um einen sicheren Halt der Ummantelung auf den Spreizelementen der Aufziehvorrichtung zu gewährleisten, wird mit der Erfindung vorgeschlagen, dass am distalen Ende der Spreizelemente ein Haltewulst zum Festlegen der Ummantelung ausgebildet ist und am proximalen Ende der Ummantelung ein umlaufender Wulst ausgebildet ist, über den die Ummantelung an den Spreizelementen festlegbar ist. Der proximalseitige umlaufende Wulst der Ummantelung dient weiterhin dazu, die Ummantelung lagesicher am distalen Ende des chirurgischen Schaftinstruments festlegen zu können. Der umlaufende Wulst dient zudem als Reißschutz für den proximalen Bereich der Ummantelung.

Schließlich wird mit der Erfindung vorgeschlagen, dass die Ummantelung aus einem elastischen Material, insbesondere Kunststoff, bestehend nur proximalseitig offen ausgebildet ist. Dabei kann die Ummantelung insbesondere als Spritzgussteil ausgebildet sein. Durch die Ausbildung der Ummantelung als nur einseitig offener Überzug aus einem elastischen Material ist es möglich, einerseits das distalseitige Arbeitswerkzeug des chirurgischen Schaftinstruments vollständig zu umschließen und andererseits aufgrund der Elastizität der Ummantelung ein Betätigen des Werkzeugs weiterhin zu ermöglichen. Weiterhin wird durch die Ummantelung die Dilatationsmechanik geschützt und ein Eindringen von Gewebe verhindert.

Weitere Merkmale und Vorteile der Erfindung ergeben sich anhand der zugehörigen Zeichnungen 1 bis 6b, in denen ein Ausführungsbeispiel einer erfindungsgemäßen Vorrichtung zum Aufbringen einer Ummantelung auf das distale Ende eines chirurgischen Schaftinstruments nur beispielhaft dargestellt ist, ohne die Erfindung auf dieses Ausführungsbeispiel zu beschränken. In den Zeichnungen zeigt:
- Fig. 1: eine perspektivische Ansicht einer ersten Ausführungsform einer erfindungsgemäßen Vorrichtung zum Aufbringen einer Ummantelung auf das distale Ende eines chirurgischen Schaftinstruments;
- Fig. 2a: eine Draufsicht auf die Vorrichtung gemäß Fig. 1, die Ausgangsstellung darstellend;
- Fig. 2b: eine teilweise geschnittene Seitenansicht der Darstellung gemäß Fig. 2a;
- Fig. 3: eine vergrößerte Darstellung des Details III gemäß Fig. 2b;
- Fig. 4a: eine Draufsicht auf die Vorrichtung gemäß Fig. 1, die Einführstellung darstellend;
- Fig. 4b: eine teilweise geschnittene Seitenansicht der Darstellung gemäß Fig. 4a;
- Fig. 5a: eine Draufsicht auf die Vorrichtung gemäß Fig. 1, die Ummantelungsstellung darstellend;
- Fig. 5b: eine Seitenansicht der Darstellung gemäß Fig. 5a;
- Fig. 6a: eine Draufsicht auf die Vorrichtung gemäß Fig. 1, die Entnahmestellung darstellend; und
- Fig. 6b: eine teilweise geschnittene Seitenansicht der Darstellung gemäß Fig. 6a;.
- Fig. 7: eine Seitenansicht einer weiteren Ausführungsform einer Vorrichtung zum Aufbringen einer Ummantelung auf das distale Ende eines chirurgischen Schaftinstruments, die Ausgangsstellung darstellend;
- Fig. 8: eine Ansicht gemäß Fig. 7, die Einführstellung darstellend und
- Fig. 9: eine Ansicht gemäß Fig. 7, die Ummantelungsstellung darstellend.

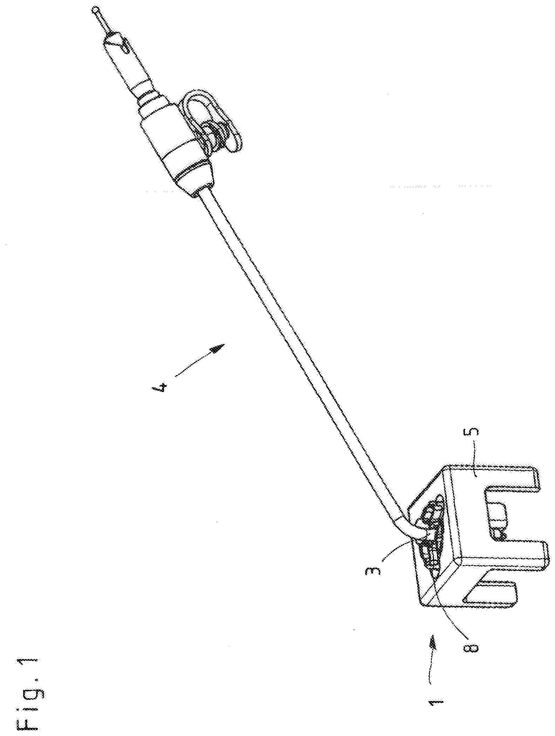

Die Abbildungen Fig. 1 bis 6b zeigen eine erste Ausführungsform einer Aufziehvorrichtung 1 zum Aufbringen einer Ummantelung 2 auf das distale Ende 3 eines chirurgischen Schaftinstruments 4.

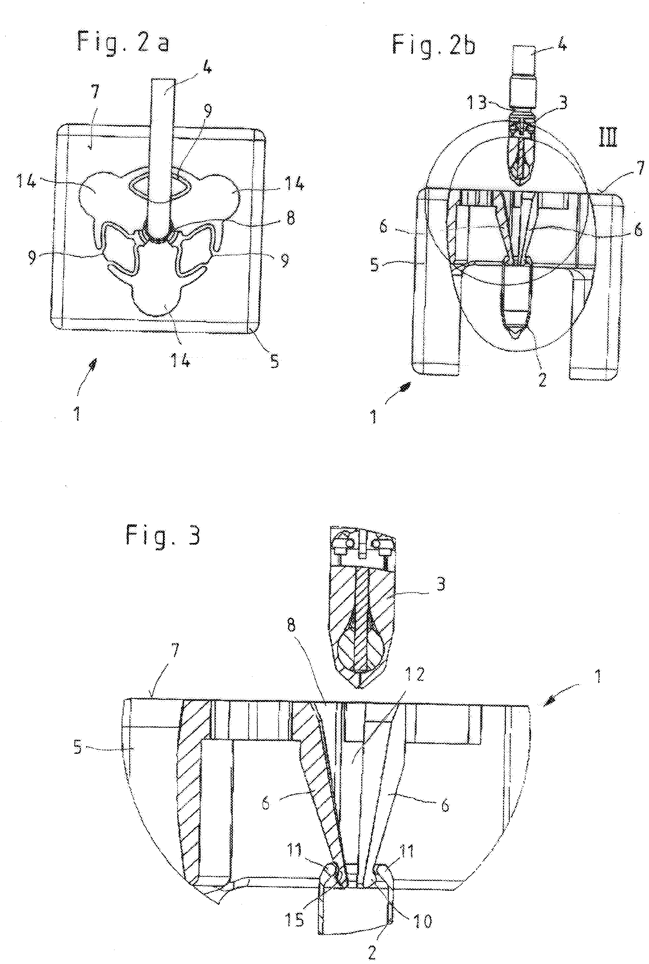

Wie insbesondere aus Fig. 3 ersichtlich, besteht die dargestellte erste Ausführungsform der Aufziehvorrichtung 1 im Wesentlichen aus einem kastenförmigen Grundkörper 5 und Spreizelementen 6, die sich von einer oberen Stirnplatte 7 des Grundkörpers 5 fort ins Innere des Grundkörpers 5 erstrecken, wobei zwischen den Spreizelementen 6 in der Stirnplatte 7 des Grundkörpers 5 eine Einführöffnung 8 zur Aufnahme des die Instrumentenspitze 3 bildenden distalen Endes 3 des chirurgischen Schaftinstruments 4 ausgebildet ist.

Bei der dargestellten Ausführungsform weist die Aufziehvorrichtung 1 drei Spreizelemente 6 auf, die mit ihren proximalen Enden kippbeweglich so an der Stirnplatte 7 gelagert sind, dass sich die distalen Enden beim Verschwenken der Spreizelemente 6 im Wesentlichen radial zur Einführöffnung 8 bewegen. Zwischen den einzelnen Spreizelementen 6 ist mindestens ein Federelement 9 angeordnet, um ein gleichmäßiges Verschwenken der Spreizelemente 6 zu gewährleisten.

Die drei Spreizelemente 6 sind bei der dargestellten Ausführungsform radial um 120° zueinander versetzt angeordnet, so dass diese Anordnung mit der Anordnung der Maulteile der zu ummantelnden Instrumentenspitze 3 übereinstimmt, wodurch ein Aufweiten der Maulteile der Instrumentenspitze 3 beim Einführen in die Aufziehvorrichtung 1 verhindert wird.

Alternativ oder zusätzlich zur Verwendung des mindestens einen Federelements 9 ist es möglich, mindestens ein Spreizelement 6 federnd auszugestalten, wobei bei der zusätzlichen Federelastizität der Spreizelemente 6 diese durch die Dimensionierung der Spreizelemente 6 und/oder durch deren Materialbeschaffenheit so einzustellen ist, dass die Federelastizität des wenigstens einen federnd ausgebildeten Spreizelements 6 geringer ausgebildet ist als die Federkraft des mindestens einen Federelements 9.

Alternativ zu der dargestellten Ausführungsform ist es auch möglich, nur zwei Spreizelemente 6 oder auch mehr als drei Spreizelemente 6 zu verwenden.

Wie insbesondere aus Fig. 32b ersichtlich, ist am distalen Ende der Spreizelemente 6 ein nach außen weisender Haltewulst 10 ausgebildet, auf dem die Ummantelung 2 an den Spreizelementen 6 festlegbar ist. Zum selben Zweck weist die Ummantelung 2 proximalseitig einen umlaufenden Wulst 11 auf, der mit dem Haltewulst 10 der Spreizelemente 6 zusammen einen sicheren Halt der Ummantelung 2 an der Aufziehvorrichtung 1 gewährleistet.

Die Detaildarstellung der Spreizelemente 6 gemäß Fig. 3 zeigt neben der zuvor beschriebenen Ausbildung der distalseitigen Haltewulst 10 zum Festlegen der umlaufenden Wulst 11 der Ummantelung 2 am rechten Spreizelement 6 eine alternative Ausgestaltungsform an dem auf der Abbildung linken Spreizelement 6. Bei dieser alternativen Ausführungsform ist am distalen Ende des Spreizelements 6 eine Halteausnehmung 15 ausgebildet, in der der proximalseitige umlaufende Wulst 11 der Ummantelung 2 festlegbar ist.

Das Aufbringen einer Ummantelung 2 auf das distale Ende 3 eines chirurgischen Schaftinstruments 4, insbesondere die Werkzeugspitze eines Dilatationsinstruments, wird für die erste Ausführungsform der Aufziehvorrichtung 1 nachfolgend anhand der Abbildungen Fig. 2a bis 6b beschrieben.

Fig. 2a bis 3 zeigen die Ausgangsstellung vor dem Aufbringen der Ummantelung 2 auf die Instrumentenspitze 3. Zunächst wird die Ummantelung 2 manuell an dem distalen Ende der Spreizelemente 6 der Aufziehvorrichtung 1 festgelegt, wie dies insbesondere der Abbildung Fig. 3 zu entnehmen ist. In dieser Ausgangsstellung hintergreift der umlaufende Wulst 11 der nur proximalseitig offenen Ummantelung 2 die Haltewulste 10 an den distalen Enden der Spreizelemente 6. Die Innenseiten der Spreizelemente 6 bilden in der Ausgangsstellung einen sich zur Einführöffnung 8 hin erweiternden trichterförmigen Einführschacht 12 für die zu ummantelnde Instrumentenspitze 3.

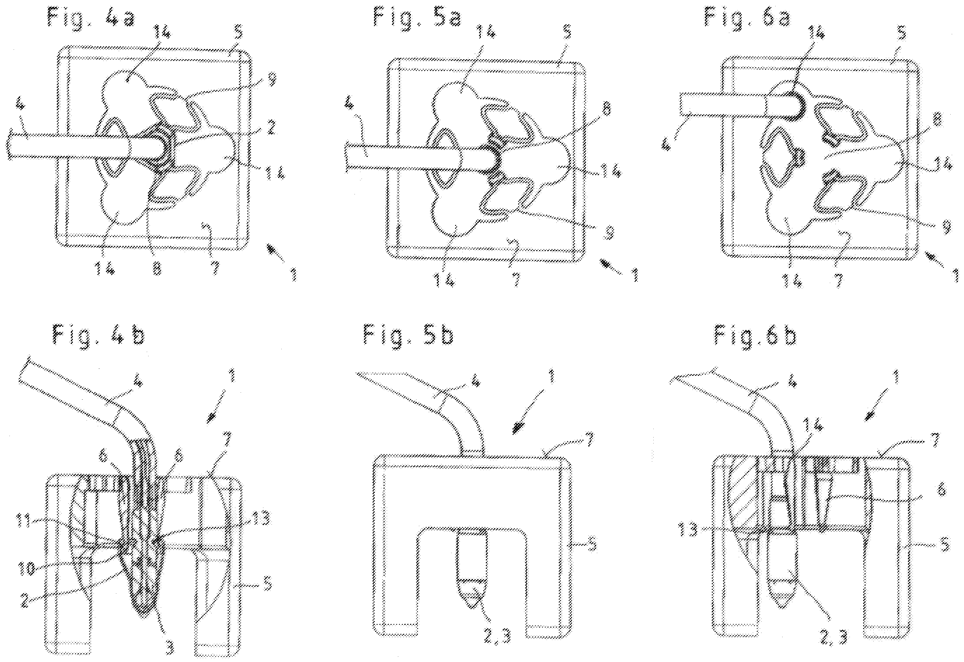

Zum Umhüllen der Instrumentenspitze 3 mit der Ummantelung 2 wird nunmehr das chirurgische Schaftinstrument 4 mit der Instrumentenspitze 3 voran senkrecht in die Einführöffnung 8 und den durch die Spreizelemente 6 gebildeten Einführschacht 12 eingeführt. Beim Einschieben der Instrumentenspitze 3 in den sich zum Inneren der Aufziehvorrichtung 1 hin verjüngenden Einführschacht 12 werden die Spreizelemente 6 durch die Instrumentenspitze 3 gegen die Kraft der Federelemente 9 radial nach außen gedrückt, was zu einem radialen Aufweiten des an den distalen Enden der Spreizelemente 6 festgelegten proximalen Endes der Ummantelung 2 führt, wie dies insbesondere der Abbildung Fig. 4b zu entnehmen ist.

In dieser in Fig. 4a und 4b dargestellten Einführstellung ist die Instrumentenspitze 3 bereits in der Ummantelung 2 angeordnet, die Ummantelung 2 selber ist aber noch über ihren proximalseitigen umlaufenden Wulst 11 an den Haltewulsten 10 der Spreizelemente 6 festgelegt.

Zum vollständigen Aufbringen der Ummantelung 2 auf die Instrumentenspitze 3 wird die Instrumentenspitze 3 senkrecht nach unten weiter in das Innere der Aufziehvorrichtung 1 gedrückt, bis der proximalseitige umlaufende Wulst 11 der Ummantelung 2 von den Haltewulsten 10 der Spreizelemente 6 herabgezogen wird und in einer umlaufenden Nut 13 am distalseitigen Ende der Instrumentenspitze 3 Aufnahme findet und so die Ummantelung 2 lagesicher auf der Instrumentenspitze 3 festlegt. Diese Ummantelungsstellung ist den Abbildungen Fig. 5a und 5b zu entnehmen.

Da beim Zurückziehen der mit der Ummantelung 2 versehenen Instrumentenspitze 3 zwischen den Spreizelementen 6 hindurch und durch die Einführöffnung 8 die Gefahr besteht, dass die Ummantelung 2 beschädigt oder heruntergezogen werden könnte, ist in der Stirnplatte 7 des Grundkörpers 5 seitlich neben der Einführöffnung 8 mindestens eine mit der Einführöffnung 8 verbundene, im Durchmesser größere Entnahmeöffnung 14 zur Entnahme der mit der Ummantelung 2 versehenen Instrumentenspitze 3 ausgebildet, wie dies den Abbildungen Fig. 6a und 6b zu entnehmen ist.

In dieser Entnahmestellung kann die mit der Ummantelung 2 versehenen Instrumentenspitze 3 nunmehr einfach und gefahrlos wieder aus der Aufziehvorrichtung 1 herausgezogen werden.

Alternativ zu der dargestellten Ausführungsform der Aufziehvorrichtung 1 mit einem kastenförmigen Grundkörper 5, an dem die Spreizelemente 6 verschwenkbar gelagert sind, ist es auch möglich, die Aufziehvorrichtung 1 zangenförmig so auszubilden, dass die distalen Enden der beiden Zangenbranchen als rechwinklig zur Längsachse der Zange abstehende Spreizelemente 6 ausgebildet sind, an denen die Ummantelung 2 festlegbar ist. Das radiale Aufweiten des proximalen Endes der Ummantelung 2 bzw. das radiale Spreizen der Spreizelemente 6 erfolgt bei dieser Ausführungsform durch das Betätigen der proximalseitigen Griffteile der Zange.

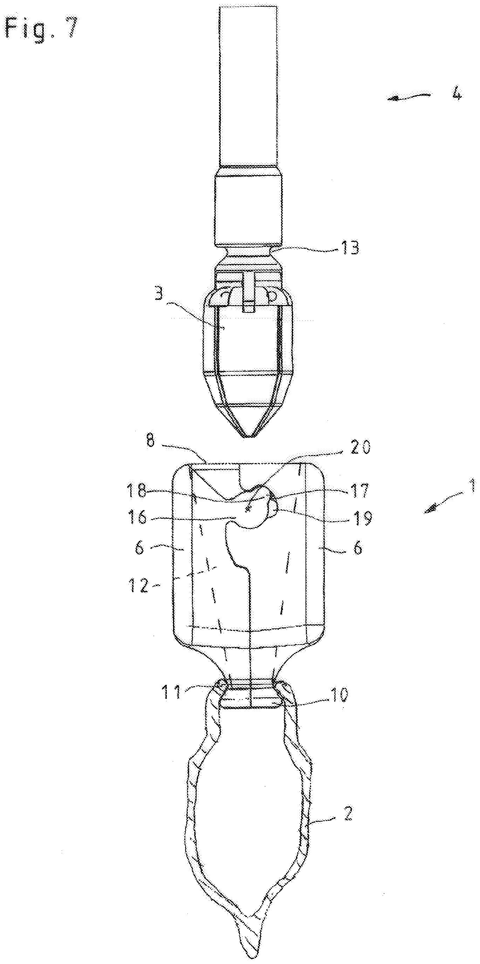

Die Abbildungen Fig. 7 bis 9 zeigen eine weitere, nicht beanspruchte Ausführungsform der Aufziehvorrichtung 1. Eine wie voranstehend beschrieben ausgebildete Vorrichtung zum Aufbringen einer Ummantelung 2 auf das distale Ende 3 eines chirurgischen Schaftinstruments 4.

Diese weitere Ausführungsform der Aufziehvorrichtung 1 unterscheidet sich von der anhand der Abbildungen Fig. 1 bis 6b beschriebenen ersten Ausführungsform im Wesentlichen dadurch, dass bei dieser Ausführungsform die Spreizelemente 6 nicht an einem Grundkörper gelagert sind, sondern unmittelbar aneinander.

Bei der dargestellten Ausführungsform besteht die Aufziehvorrichtung 1 aus zwei im Querschnitt U-förmig halbschalig ausgebildeten Spreizelementen 6, die gegeneinander verschwenkbar aneinander gelagert sind.

Zum gegenseitigen Verschwenken und gegenseitigen aneinander Lagern sind an den Spreizelementen 6 Lagerzapfen 16 und Lageraufnahmen 17 ausgebildet, die ineinander greifen und einander hintergreifen. Vorteilhafterweise sind an jedem Spreizelement 6 ein Lagerzapfen 16 und eine Lageraufnahme 17 ausgebildet, so dass die beiden die Aufziehvorrichtung 6 bildenden Spreizelemente 6 eine umgekehrt gespiegelte Geometrie aufweisen. Aufgrund dieser Ausgestaltung bedarf nur der Ausbildung eines Spreizarmtyps, um ohne linke oder rechte Halbschale vertauschen zu können, aus zwei Spreizelementen 6 eine Aufziehvorrichtung 1 zusammensetzen zu können.

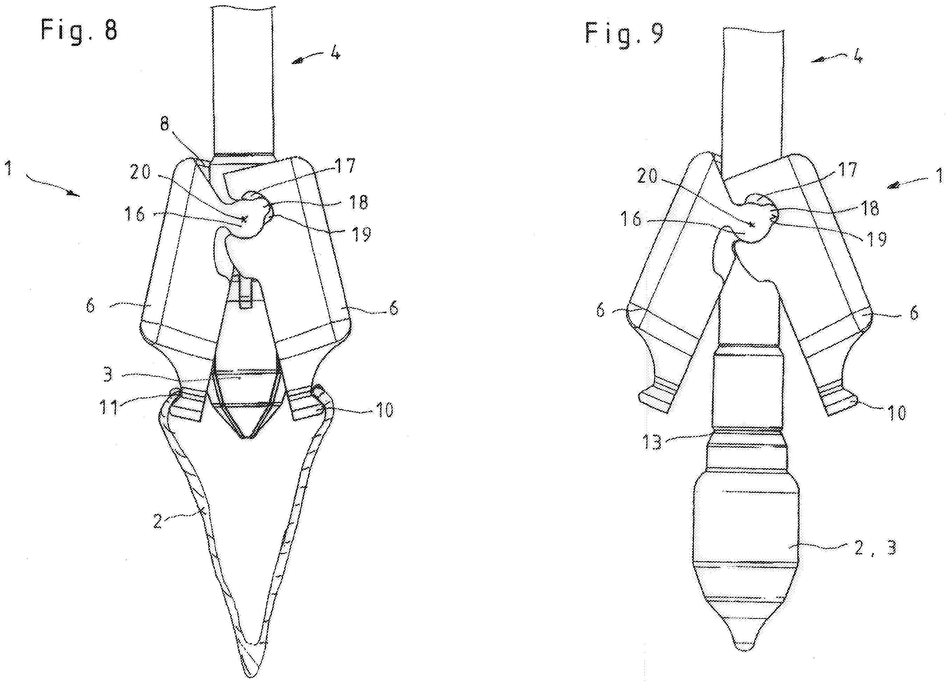

Zur Montage der Aufziehvorrichtung 1 sowie zum Abnehmen der Aufziehvorrichtung 1 von der ummantelten Instrumentenspitze 3 sind die Spreizelemente 6 voneinander lösbar ausgebildet. Hierzu sind die Lagerzapfen 16 und die Lageraufnahmen 17 so ausgebildet sind, dass die Spreizelemente 6 nur in einer einzigen gegeneinander verschwenkten Position voneinander lösbar sind. Vorteilhafterweise ist dies die in Fig. 9 dargestellte, am weitesten gegeneinander verschwenkte Position der Spreizelemente 6, um ein versehentliches Lösen der Spreizelemente 6 während der Ummantelungsphase zu vermeiden.

Um dieses Lösen in nur einer bestimmten verschwenkten Position der Spreizelemente 6 zu ermöglichen, ist bei der dargestellten Ausführungsform an jedem Lagerzapfen 16 ein Vorsprung 18 angeformt, der in der nicht verschwenkten oder nur teilweise verschwenkten Position der Spreizelemente 6 das Herausziehen des jeweiligen Lagerzapfens 16 aus der Lageraufnahme 17 verhindert. In jeder Lageraufnahme 17 ist eine Erweiterung 19 ausgebildet, in die der jeweilige Vorsprung 18 des Lagerzapfens 16 ausschließlich in der vorgesehenen Lösestellung so eingreift, dass die Spreizelemente 6 voneinander getrennt werden können.

Das Aufbringen einer Ummantelung 2 auf das distale Ende 3 eines chirurgischen Schaftinstruments 4, insbesondere die Werkzeugspitze eines Dilatationsinstruments, wird für die zweite Ausführungsform der Aufziehvorrichtung 1 nachfolgend anhand der Abbildungen Fig. 7 bis 9 beschrieben.

Fig. 7 zeigt die Ausgangsstellung vor dem Aufbringen der Ummantelung 2 auf die Instrumentenspitze 3. Zunächst wird die Ummantelung 2 manuell an den distalen Ende der Spreizelemente 6 der Aufziehvorrichtung 1 festgelegt, wie dies insbesondere der Abbildung Fig. 7 zu entnehmen ist. In dieser Ausgangsstellung hintergreift der umlaufende Wulst 11 der nur proximalseitig offenen Ummantelung 2 die Haltewulste 10 an den distalen Enden der Spreizelemente 6. Die Innenseiten der Spreizelemente 6 bilden in der Ausgangsstellung einen sich zur Einführöffnung 8 hin erweiternden trichterförmigen Einführschacht 12 für die zu ummantelnde Instrumentenspitze 3.

Zum Umhüllen der Instrumentenspitze 3 mit der Ummantelung 2 wird nunmehr das chirurgische Schaftinstrument 4 mit der Instrumentenspitze 3 voran senkrecht in die Einführöffnung 8 und den durch die Spreizelemente 6 gebildeten Einführschacht 12 eingeführt. Beim Einschieben der Instrumentenspitze 3 in den sich zum Inneren der Aufziehvorrichtung 1 hin verjüngenden Einführschacht 12 werden die Spreizelemente 6 um eine durch die in den Lageraufnahmen 17 gelagerten Lagerzapfen 16 gebildete Schwenkachse 20 distalseitig gegeneinander nach außen verschwenkt, was zu einem radialen Aufweiten des an den distalen Enden der Spreizelemente 6 festgelegten proximalen Endes der Ummantelung 2 führt, wie dies der Abbildung Fig. 8 zu entnehmen ist.

In dieser in Fig. 8 dargestellten Einführstellung ist die Instrumentenspitze 3 bereits teilweise in der Ummantelung 2 angeordnet, die Ummantelung 2 selber ist aber noch über ihren proximalseitigen umlaufenden Wulst 11 an den Haltewulsten 10 der Spreizelemente 6 festgelegt.

Zum vollständigen Aufbringen der Ummantelung 2 auf die Instrumentenspitze 3 wird die Instrumentenspitze 3 senkrecht nach unten weiter in das Innere der Aufziehvorrichtung 1 gedrückt, bis der proximalseitige umlaufende Wulst 11 der Ummantelung 2 von den Haltewulsten 10 der Spreizelemente 6 herabgezogen wird und in einer umlaufenden Nut 13 am distalseitigen Ende der Instrumentenspitze 3 Aufnahme findet und so die Ummantelung 2 lagesicher auf der Instrumentenspitze 3 festlegt. Diese Ummantelungsstellung ist der Abbildung Fig. 9 zu entnehmen.

In dieser Ummantelungstellung, in der die Spreizelemente 6 am weitesten gegeneinander verschwenkt sind, können die Spreizelemente 6 durch Herausziehen der Lagerzapfen 16 aus den Lageraufnahmen 17 voneinander getrennt und von der ummantelten Instrumentenspitze 3 abgenommen werden.

Die wie voranstehend beschrieben ausgebildeten Vorrichtungen zum Aufbringen einer Ummantelung 2 auf das distale Ende 3 eines chirurgischen Schaftinstruments 4 zeichnen zeichnet sich dadurch aus, dass es bei einfacher Handhabung und einfachem Aufbau der Aufziehvorrichtung 1 möglich ist, die Ummantelung 2 schnell und ohne die Gefahr einer Beschädigung der Ummantelung 2 auf der Instrumentenspitze 3 eines chirurgischen Schaftinstruments 4 anzuordnen.

**Bezugszeichenliste**

| | | | |
|---|---|---|---|
| 1 | Aufziehvorrichtung | 18 | Vorsprung |
| 2 | Ummantelung | 19 | Erweiterung |
| 3 | distales Ende / Instrumentenspitze | 20 | Schwenkachse |
| 4 | chirurgisches Schaftinstrument | | |
| 5 | Grundkörper | | |
| 6 | Spreizelement | | |
| 7 | Stirnplatte | | |
| 8 | Einführöffnung | | |
| 9 | Federelement | | |
| 10 | Haltewulst | | |
| 11 | Wulst | | |
| 12 | Einführschacht | | |
| 13 | Nut | | |
| 14 | Entnahmeöffnung | | |
| 15 | Halteausnehmung | | |
| 16 | Lagerzapfen | | |
| 17 | Lageraufnahme | | |

## Patentansprüche

1. Vorrichtung zum Aufbringen einer Ummantelung auf das distale Ende eines chirurgischen Schaftinstruments,
mit einem mindestens zwei Spreizelemente (6) aufweisenden Grundkörper (5), wobei die Ummantelung (2) an den Spreizelementen (6) festlegbar ist und zwischen den Spreizelementen (6) eine Einführöffnung (8) zur Aufnahme des distalen Endes (3) des chirurgischen Schaftinstruments (4) ausgebildet ist,
wobei die mindestens zwei Spreizelemente (6) radial zur Einführöffnung (8) verlagerbar am Grundkörper (5) gelagert sind, wobei wenigstens ein Spreizelement (6) federnd ausgebildet ist,
**dadurch gekennzeichnet, dass** die Vorrichtung mindestens ein Federelement (9) umfasst und die Spreizelemente (6) gegen die Kraft dieses mindestens einen Federelements radial verschwenkbar sind.

2. Vorrichtung nach Anspruch 1, **dadurch gekennzeichnet, dass** der Grundkörper (5) kastenförmig so ausgebildet ist, dass die Einführöffnung (8) in einer Stirnplatte (7) des Grundkörpers (5) ausgebildet ist und sich die Spreizelemente (6) von der Einführöffnung (8) fort in das Innere des Grundkörpers (5) erstrecken.

3. Vorrichtung nach den Anspruch 1, **dadurch gekennzeichnet, dass** die Federelastizität des wenigstens einen federnd ausgebildeten Spreizelements (6) geringer ausgebildet ist als die Federkraft des mindestens einen Federelements (9).

4. Vorrichtung nach einem der Ansprüche 1 bis 3, **dadurch gekennzeichnet, dass** in der Stirnplatte (7) des Grundkörpers (5) seitlich neben der Einführöffnung (8) mindestens eine mit der Einführöffnung (8) verbundene Entnahmeöffnung (14) zur Entnahme der mit der Ummantelung (2) versehenen Instrumentenspitze (3) ausgebildet ist.

5. Vorrichtung nach einem der Ansprüche 1 bis 4, **dadurch gekennzeichnet, dass** der Grundkörper (5) und/oder die Spreizelemente (6) als Spritzgußteile ausgebildet sind.

6. Vorrichtung nach einem der Ansprüche 1 bis 5, **dadurch gekennzeichnet, dass** am distalen Ende der Spreizelemente (6) ein Haltewulst (10) oder eine Halteausnehmung (15) zum Festlegen der Ummantelung (2) ausgebildet ist.

7. Vorrichtung nach einem der Ansprüche 1 bis 6, **dadurch gekennzeichnet, dass** die Vorrichtung eine Ummantelung umfasst, wobei am proximalen Ende der Ummantelung (2) ein umlaufender Wulst (11) ausgebildet ist, über den die Ummantelung (2) an den Spreizelementen (6) und am distalen Ende (3) des chirurgischen Schaftinstruments (4) festlegbar ist.

## Claims

1. Device for applying a sheath to the distal end of a surgical shaft instrument,
with a main body (5) having at least two spreading elements (6), wherein the sheath (2) can be fastened to the spreading elements (6) and an insertion opening (8) for receiving the distal end (3) of the surgical shaft instrument (4) is formed between the spreading elements (6),
wherein the at least two spreading elements (6) are mounted on the main body (5) such that they can be displaced radially in relation to the insertion opening (8), wherein at least one spreading element (6) is of resilient form,
**characterized in that** the device comprises at least one spring element (9) and the spreading elements (6) can be pivoted radially against the force of said at least one spring element.

2. Device according to Claim 1, **characterized in that** the main body (5) is of box-shaped form in such a way that the insertion opening (8) is formed in an end plate (7) of the main body (5) and the spreading elements (6) extend from the insertion opening (8) into the interior of the main body (5).

3. Device according to Claim 1, **characterized in that** the spring elasticity of the at least one spreading element (6) of resilient form is configured to be lower than the spring force of the at least one spring element (9).

4. Device according to one of Claims 1 to 3, **characterized in that** at least one removal opening (14) connected to the insertion opening (8) is formed in the end plate (7) of the main body (5), laterally alongside the insertion opening (8), for removing the instrument tip (3) provided with the sheath (2).

5. Device according to one of Claims 1 to 4, **characterized in that** the main body (5) and/or the spreading elements (6) are in the form of injection-moulded parts.

6. Device according to one of Claims 1 to 5, **characterized in that** a retaining bead (10) or a retaining recess (15) for fastening the sheath (2) is formed on the distal end of the spreading elements (6).

7. Device according to one of Claims 1 to 6, **characterized in that** the device comprises a sheath, wherein a circumferential bead (11) is formed on the proximal end of the sheath (2), by way of which bead the sheath (2) can be fastened to the spreading elements (6) and to the distal end (3) of the surgical shaft instrument (4).

## Revendications

1. Dispositif d'application d'une gaine sur l'extrémité distale d'un instrument chirurgical à tige,
avec un corps de base (5) présentant au moins deux éléments expansibles (6), dans lequel la gaine (2) peut être fixée sur les éléments expansibles (6) et une ouverture d'introduction (8) est formée entre les éléments expansibles (6) afin de recevoir l'extrémité distale (3) de l'instrument chirurgical à tige (4),
dans lequel lesdits au moins deux éléments expansibles (6) sont montés sur le corps de base (5) de façon déplaçable radialement par rapport à l'ouverture d'introduction (8), dans lequel au moins un élément expansible (6) est réalisé sous forme élastique, **caractérisé en ce que** le dispositif comprend au moins un élément de ressort (9) et les éléments expansibles (6) peuvent pivoter radialement contre la force dudit au moins un élément de ressort.

2. Dispositif selon la revendication 1, **caractérisé en ce que** le corps de base (5) est réalisé en forme de caisse de telle manière que l'ouverture d'introduction (8) soit formée dans une plaque frontale (7) du corps de base (5) et que les éléments expansibles (6) se prolongent à partir de l'ouverture d'introduction (8) jusqu'à l'intérieur du corps de base (5).

3. Dispositif selon la revendication 1, **caractérisé en ce que** l'élasticité dudit au moins un élément expansible (6) réalisé sous forme élastique est inférieure à la force élastique dudit au moins un élément de ressort (9).

4. Dispositif selon l'une quelconque des revendications 1 à 3, **caractérisé en ce qu'**au moins une ouverture d'enlèvement (14) reliée à l'ouverture d'introduction (8) est formée dans la plaque frontale (7) du corps de base (5) latéralement à côté de l'ouverture d'introduction (8) pour l'enlèvement de la pointe (3) de l'instrument munie de la gaine (2).

5. Dispositif selon l'une quelconque des revendications 1 à 4, **caractérisé en ce que** le corps de base (5) et/ou les éléments expansibles (6) sont formés par des pièces moulées par injection.

6. Dispositif selon l'une quelconque des revendications 1 à 5, **caractérisé en ce qu'**un bourrelet de retenue (11) ou un évidement de retenue (15) est formé(e) sur l'extrémité distale des éléments expansibles (6) pour la fixation de la gaine (2).

7. Dispositif selon l'une quelconque des revendications 1 à 6, **caractérisé en ce que** le dispositif comprend une gaine, dans lequel un bourrelet périphérique (11) est formé sur l'extrémité proximale de la gaine (2), par lequel la gaine (2) peut être fixée sur les éléments expansibles (6) et sur l'extrémité distale (3) de l'instrument chirurgical à tige (4).
